(19) **Europäisches Patentamt / European Patent Office / Office européen des brevets**

(11) **EP 4 501 288 A1**

(12) # EUROPEAN PATENT APPLICATION
published in accordance with Art. 153(4) EPC

(43) Date of publication:
05.02.2025  Bulletin 2025/06

(21) Application number: 23818841.1

(22) Date of filing: 25.04.2023

(51) International Patent Classification (IPC):
A61F 5/01 (2006.01)       A61H 1/02 (2006.01)
A61B 5/369 (2021.01)      A61B 5/0205 (2006.01)
A61B 5/145 (2006.01)      A61B 5/107 (2006.01)
G01G 19/50 (2006.01)      A61B 5/388 (2021.01)

(86) International application number:
PCT/CN2023/090570

(87) International publication number:
WO 2023/236674 (14.12.2023 Gazette 2023/50)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 09.06.2022  CN 202210650783

(71) Applicant: Wuhan Klarity Medical Tech Co., Ltd.
Wuhan, Hubei 430070 (CN)

(72) Inventors:
• LAN, Peiqin
  Guangzhou, Guangdong 510730 (CN)
• WANG, Bangde
  Wuhan, Hubei 430070 (CN)
• LI, Jinhua
  Wuhan, Hubei 430070 (CN)
• LIU, Jinsong
  Wuhan, Hubei 430070 (CN)

(74) Representative: Lavoix
Bayerstraße 83
80335 München (DE)

(54) **INTELLIGENT SCOLIOSIS REHABILITATION ROBOT WITH CLOUD STORAGE FUNCTION**

(57) Provided is an intelligent scoliosis rehabilitation robot with a cloud storage function, which comprises: a head supporting electroencephalogram and near-infrared monitoring module, used for supporting the head of a scoliosis patient in the spine correction process, monitoring the electroencephalogram, pulse, blood oxygen saturation and light nerve fast signal of the scoliosis patient, and adjusting and correcting force application; a scoliosis posture detection module, used for detecting a scoliosis state and a body type state of the scoliosis patient; a specific parameter cloud analysis module, used for transmitting a scoliosis posture detection result and medical examination data to a cloud storage analysis system, and automatically generating specific adaptive parameters of the scoliosis patient; and a program control and execution robot module, used for importing specific individualized parameters into a scoliosis rehabilitation control program, and controlling a rehabilitation robot execution mechanism to carry out specific intelligent rehabilitation correction on the scoliosis patient. The rehabilitation robot can provide an accurate three-dimensional correction force for multidirectional stress correction treatment on a patient, feeds back the treatment process and effect in real time, guides follow-up treatment and the design of a daily wearing support, and can provide a treatment result for other patients to serve as a treatment reference.

**FIG. 1**

## Description

The present application claims the priority to Chinese Patent Application No. 202210650783.2, titled "INTELLIGENT SCOLIOSIS REHABILITATION ROBOT WITH CLOUD STORAGE FUNCTION", filed with the China National Intellectual Property Administration on June 9, 2022, the entire disclosure of which is incorporated herein by reference.

## FIELD

The present application relates to the technical field of intelligent scoliosis rehabilitation robots, and in particular to an intelligent scoliosis rehabilitation robot with a cloud storage function.

## BACKGROUND

In a conventional scoliosis treatment device, the number of degrees of freedom of a push arm is generally limited, or the manual adjustment to the push arm is required, which limits the combinations of the push forces, and also increases workloads of medical staffs. Scoliosis, or sideways curvature of the spine, refers to spine deformity over three dimensions with sideways curvature of one or multiple segments of the spine, or with vertebral rotation at the same time, including abnormalities of the spinal column in the coronal, sagittal and horizontal planes. A normal spine should be a straight line in a back view, and the trunk should be left-right symmetrical. Mild scoliosis generally does not cause significant discomfort, and no apparent body deformity can be seen. Severe scoliosis can affect the growth and development of infants, children, or adolescents, and deform the body. More severe scoliosis can affect cardiopulmonary functions, and even affect the spinal cord to cause paralysis. Mild scoliosis may be observed, and moderate cases need timely rehabilitation treatments, while severe scoliosis requires a surgical treatment.

Scoliosis often occurs in adolescents, and has an incidence rate of 2% to 3% around the globe. In China, the incidence rate of scoliosis is up to 5%. The Cobb angle is often used to clinically describe the severity of scoliosis. A certain severity of scoliosis (where the Cobb angle is between 20 degrees and 40 degrees) may select conservative treatments. The degree of scoliosis can be reduced by a force applied on a scoliosis position for a period of time by certain means. Both a continuous push force and an intermittent push force can bring treatment outcomes. According to existing solutions (such as Patent No. CN112168452A, titled "THREE-DIMENSIONAL SCOLIOSIS PUSHING FORCE CORRECTION DEVICE AND APPLICATION THEREOF", Patent No. CN215081530U, titled "AUXILIARY DEVICE FOR SCOLIOSIS TREATMENT", Patent No. CN114041910A, titled "ARTIFICIAL-INTELLIGENCE SCOLIOSIS TREATMENT DEVICE AND USING METHOD THEREOF", Patent No. CN109172082A, titled "THREE-DIMENSIONAL SCOLIOSIS CORRECTIVE TRAINING FRAME", Patent No. CN113974937A, titled "SCOLIOSIS CORRECTION DEVICE", Patent No. CN213310693U, titled "AUXILIARY DEVICE FOR SCOLIOSIS TREATMENT", and Patent No. CN210990977U, titled "TARGETED PRESSURE DEVICE FOR SCOLIOSIS CORRECTION"), a treatment bed or a frame style treatment device is usually adopted. First, a patient should lie on the bed or sit on a chair firstly, and some parts of the body, such as the pelvis and armpits, are fixed. Then, corrective push arms apply forces on different positions of scoliosis with manual, electric, or pneumatic power sources, so that the scoliosis is alleviated to achieve treatment purpose. After the treatment, the patient is provided with a brace for use at home. In an existing device, push arms are generally provided at two to four points along the coronal plane, i.e. the push arms apply forces at left and right sides of the patient for treatment. For example, that one push arm is placed at a convex side and two push arms are placed at a concave side can exert a valuable force set. If the spine of the patient is curved in an opposite direction, the patient may change direction, and a similar three-point force application solution can still be used for treatment.

The conventional technology has following problems.

For the treatment of C-shaped scoliosis, the three-point force application can bring a relatively good treatment outcome. However, for S-shaped scoliosis, three push arms may be insufficient for treatments of scoliosis occurring at two or more positions. Therefore, more push arms are needed to meet both cases of patients with the C-shaped scoliosis and the S-shaped scoliosis. Moreover, the scoliosis is not limited within the coronal plane. If the push arms are mounted on a fixed frame, or only have few degrees of freedom including up-down and left-right movements, there is a lack of a better pushing solution for scoliosis in a three-dimensional space. That is, a more scientific method is expected, so that the forces applied by the push arms have more degrees of freedom, including up-down movements and rotations around a vertical axis. In existing solutions employing a multi-degree-of-freedom pushing correction device, either the direction of the correction device is fixed and the degrees of freedom are limited, or the direction of the correction device is manually adjusted to meet the requirements for force application in different directions. These processes which involve complete or partial manual adjustments are time-consuming and cumbersome to get outcomes. In the conventional treatment device, the push arm has insufficient degrees of freedom, or the push arm is need to be manually adjusted, which limits the combinations of the push forces, and increases the workloads of the medical staffs. A more automated multi-degree-of-freedom robot is demanded for providing rehabilitation treatment to the patients. With the automated adjustment process, the operation efficiency of the solution can be improved.

In addition, when the patient sits on the chair for scoliosis treatment, the pelvis of the patient should be fixed for better treatment. Furthermore, the pelvises of some patients may incline itself in a front-back direction, a left-right direction or even more directions. Therefore, the seat is expected to be inclinable after the pelvis of the patient is fixed, so that the scoliosis treatment can be performed on the patient after the pelvis of the patient is adjusted to a normal anatomical position, so as to achieve better correction treatment results. According to the existing solutions, the chair can only fix the pelvis of the patient, or be inclinable in the left-right direction. Actually, the pelvis may incline in any direction, which is not limited to the left-right direction.

In a conventional scoliosis treatment device, the combinations of the push forces are limited, and workloads for the medical staffs are increased. A more automated multi-degree-of-freedom robot is demanded for providing rehabilitation treatment to the patients. During the treatment process, some clinicians do measurements on the back of the patient to obtain the scoliosis correction results. Thus, the treatment device is expected to allow the back of the patient to be exposed, and provide a space for the clinicians to operate an imaging device to monitor scoliosis parameters such as a Cobb angle. Existing bed-type and ring-type solutions cannot meet this clinical demand well.

In the existing solutions, the patient being treated is aware of nothing about the treatment process, or about the body status to be achieved. If the patient can know the current treatment stage and the correction results by watching or hearing, the treatment outcomes on the patient can be mentally improved.

In addition, during the treatment process of the patient, it is mainly the medical staff that monitors the entire treatment process, accompanies the patient, and takes corresponding actions in case of an emergency, which increases the workloads of the medical staff. Moreover, patient health parameters in the entire treatment process are not monitored. If physiological parameters of the patient such as an electroencephalogram, a pulse rate, oxygen saturation, heart rate variability, a fast optical neuronal signal and the like can be monitored at the same time, the physiological parameters can provide a data source for objective evaluation of unexpected physical conditions. Furthermore, under the monitoring of the physiological parameters, feelings of the patient during the treatment process can be objectively obtained, and the treatment can be adjusted in time to enhance the treatment outcomes.

The treatment of scoliosis is a comprehensive project. Some patients need surgical treatments, and other patients need continual home treatments or to wear a brace. However, the design of the brace usually depends on the experience of clinicians. If a force combination corresponding to an optimized treatment result during the treatment process by the scoliosis rehabilitation robot can be taken as a reference, the design of the brace can be more scientific and reasonable. There is no proper way to transmit data of the force combination of the scoliosis rehabilitation robot to the brace designer now. A more automated multi-degree-of-freedom robot is expected for providing rehabilitation treatment to the patient. Therefore, an intelligent scoliosis rehabilitation robot with a cloud storage function is expected to be provided to solve at least a part of the problems in the conventional technology.

## SUMMARY

A series of simplified concepts are introduced in the summary section, and are further described in detail in the detailed description of the embodiments section. The summary section of the present application are not intended to define the key features and essential technical features of the technical solution claimed for protection or determine the scope of protection of the technical solution claimed for protection.

To solve at least a part of the above problem, an intelligent scoliosis rehabilitation robot with a cloud storage function is provided according to the present application, including a head-support electroencephalogram and near-infrared monitoring module, a scoliosis body shape status measurement module, an individualized-parameter cloud analysis module, and a program-control and robotics actuator module,

the head-support electroencephalogram and near-infrared monitoring module is configured to support a head of a patient with scoliosis during a spinal correction process, monitor physiological signals, including an electroencephalogram, a pulse rate, oxygen saturation, and a fast optical neuronal signal, of the patient, and assist an adjustment to a corrective force;

the scoliosis body shape status measurement module is configured to measure a scoliosis status and a body shape status of the patient;

the individualized-parameter cloud analysis module is configured to transmit scoliosis body shape status measurement result and medical examination data to a cloud storage analysis system, and automatically generate an individualized adaptive parameter of the patient; and

the program-control and robotics actuator module is configured to import an individualized adaptive parameter into a scoliosis rehabilitation control program, and control an actuator mechanism of the rehabilitation robot to perform intelligent individualized rehabilitation correction on the patient.

Preferably, the head-support electroencephalogram and near-infrared monitoring module includes a head-support suspended lifting sub-module, an electroencephalogram acquisition electrode sub-module, a near-infrared monitoring sensor module, and an electrode and near-infrared-monitoring-sensor connection monitoring and tracking sub-module,

the head-support suspended lifting sub-module is configured to support the head of the patient through a head traction device during the spinal correction process, and acquire traction force data through a traction force sensor of the head traction device;

the electroencephalogram acquisition electrode sub-module is configured to acquire the electroencephalogram of the patient through a signal acquisition electrode;

the near-infrared monitoring sensor module is configured to acquire the pulse rate, the oxygen saturation, and the fast optical neuronal signal of the patient through an optical sensor; and

the electrode and near-infrared-monitoring-sensor connection monitoring and tracking sub-module is configured to assist to analyze whether a treatment process is proper according to the electroencephalogram, the pulse rate, the oxygen saturation, the fast optical neuronal signal and a brain physiological signal of the patient, and monitor an emergency where the patient is not able to call for help, such that a doctor monitors a status of the patient through the electroencephalogram, the pulse rate, the oxygen saturation, the fast optical neuronal signal and the brain physiological signal, and further adjusts the corrective force.

Preferably, the scoliosis body shape status measurement module includes a scoliosis status measurement sub-module, a patient body shape status measurement sub-module, and a rehabilitation status monitoring and measurement sub-module,

the scoliosis status measurement sub-module is configured to read a scoliosis medical image, and assist the doctor to measure the scoliosis status;

the patient body shape status measurement sub-module is configured to measure the body shape status of the patient, and measure a height, a weight, a body mass index, a subcutaneous fat thickness, and body measurements data; and

the rehabilitation status monitoring and measurement sub-module is configured to monitor a treatment status of the patient and measure a treatment outcome.

Preferably, the individualized-parameter cloud analysis module includes a cloud-platform and data-transmission sub-module, a medical examination data storage sub-module, and a cloud parameter analysis and storage sub-module, where

the cloud-platform and data-transmission sub-module is configured to upload scoliosis body shape status measurement data to a cloud, to provide a reference and a guide for a treatment of the patient and other patients by the scoliosis rehabilitation robot, and for a subsequent home treatment of the patient, and bracing design and adjustment;

the medical examination data storage sub-module is configured to transmit the medical examination data, and store the scoliosis body shape status measurement data and the medical examination data to the cloud; and

the cloud parameter analysis and storage sub-module is configured to perform cloud analysis on the scoliosis body shape status measurement data and the medical examination data, and automatically generate the individualized adaptive parameter of the patient.

Preferably, the program-control and robotics actuator module includes a cloud data import program sub-module, a scoliosis rehabilitation program control sub-module, and a control and robotics actuator sub-module, where

the cloud data import program sub-module is configured to import the individualized adaptive parameter and cloud storage data into a control unit, where the control unit includes a storage unit, a network transmission unit, and an industrial control hardware suite, and the individualized adaptive parameter and the cloud storage data are configured to be imported into the control unit through the network transmission unit, and are stored in a local storage unit;

the scoliosis rehabilitation program control sub-module is configured to read the individualized adaptive parameter in the local storage unit, input the individualized adaptive parameter into a scoliosis rehabilitation program in the industrial control hardware suite, and generate an individualized adaptive scoliosis rehabilitation program; and

the control and robotics actuator sub-module is configured to control the scoliosis rehabilitation robot to perform a scoliosis rehabilitation treatment process through the individualized adaptive scoliosis rehabilitation program, start the scoliosis rehabilitation control program, and control the actuator mechanism of the scoliosis rehabilitation robot to perform the intelligent individualized rehabilitation correction on the patient.

Preferably, the control and robotics actuator sub-module includes a scoliosis correction mechanism, a multi-angle pelvis fixation mechanism, a bearing-frame and hand-guard mechanism, and a dual-touch-screen display mechanism, where

the scoliosis correction mechanism is configured to provide a three-dimensional push force for scoliosis correction of the patient to realize a multi-degree-of-freedom automated force treatment in a three-dimensional space;

the multi-angle pelvis fixation mechanism is configured to automatically fix a pelvis of the patient, adjust a position of the pelvis of the patient, and perform adjustments in multiple inclination angles;

the bearing-frame and hand-guard mechanism is configured to serve as a hand guard during a scoliosis rehabilitation process, such that the patient puts hands onto the hand guard during the treatment process to alleviate fatigue for keeping receiving treatment during a required time period in the treatment process; and

the dual-touch-screen display mechanism is configured for a touch screen operation and for displaying a status of the treatment process through the dual-touch-screen display mechanism facing both the patient and a medical staff.

Preferably, the scoliosis correction mechanism includes a lifting module unit, a multi-axis robotic arm set unit, a scoliosis correction arm, and an image acquisition module, where

the lifting module unit is configured to drive a robotic arm of the scoliosis rehabilitation robot to move upwards and downwards, where the lifting module unit includes at least two lifting modules, and each of the at least two lifting modules includes a lifting motor set and a limit sensor set;

the multi-axis robotic arm set unit is configured to drive the scoliosis correction arm to move along a horizontal direction and push the scoliosis correction arm, where

the multi-axis robotic arm set unit includes at least four multi-axis robotic arm sets, and each of the at least four multi-axis robotic arm sets include a mechanical rotation shaft set and a force sensor set;

the mechanical rotation shaft set is connected and mounted to the at least two lifting modules, and the force sensor set includes multiple force sensors and is mounted at a connection position of a rotation pivot and a connecting rod of the mechanical rotation shaft set;

the multi-axis robotic arm set has rotation axes along an X direction, a Y direction, and a Z direction over three dimensions in a space, and is configured to drive the scoliosis correction arm such that the scoliosis correction arm can apply a force at any position of a trunk of the patient, and a direction of a push force moves upwards and downwards along a vertical axis or rotates around the vertical axis, to provide the three-dimensional push force to the patient for correction to realize the multi-degree-of-freedom automated force treatment in the three-dimensional space; and

the multi-axis robotic arm set is configured to control the robotic arm to automatically move upwards and downwards along the vertical axis or rotate around the vertical axis and provide a multi-degree-of-freedom corrective force through a setting made by a medical stuff on an interface of the dual-touch-screen display mechanism in a medical operation;

the scoliosis correction arm is configured to be driven by the robotic arm to be located at a position of a correction treatment of the patient to perform the scoliosis correction; and

the image acquisition module is configured to acquire images of the treatment process from multiple perspectives through an angle-adjustable camera.

Preferably, the multi-angle pelvis fixation mechanism includes a multi-degree-of-freedom seat unit that is used for a multi-posture force corrective treatment of the patient, where

the multi-degree-of-freedom seat unit includes a seat motor set, a seat clamping-board motor module, a seat motor limit sensor set, a seat clamping-board motor limit sensor set, and a seat clamping-board force sensor set;

the seat motor set includes a seat-adjustment drive motor and at least three seat-adjustment support electric cylinders, and the at least three seat-adjustment support electric cylinders are fixedly connected to a rotation shaft of the seat-adjustment drive motor through a bolt;

the multi-posture force corrective treatment includes force corrective treatments in a sitting posture, a standing posture, and a reclining posture;

a seat surface of the multi-degree-of-freedom seat unit is composed of a pull-out multi-layer board, an outer board of the pull-out multi-layer board is connected to the at least three seat-adjustment support electric cylinders, an inner board of the pull-out multi-layer board is connected to the outer board through a slide rail, and in a case that the inner board of the pull-out multi-layer board is pulled out, the seat surface extends to form a bed-shaped board surface, and the bed-shaped board surface is used for the force corrective treatment of the patient in the reclining posture through an adjustment to an angle of the bed-shaped board surface;

in a case that the multi-degree-of-freedom seat unit is removed from a treatment position, the corrective treatment of the patient in the standing posture is performed;

the multi-degree-of-freedom seat unit is provided with a pelvis fixation clamping board that is configured to be driven by the seat clamping-board motor module to fix the pelvis of the patient;

the at least three seat-adjustment support electric cylinders are configured to support the multi-degree-of-freedom seat unit to make the multi-degree-of-freedom seat unit move upwards and downwards or be inclined with one or multiple degrees of freedom, such that the multi-degree-of-freedom seat unit is inclined by an angle towards any direction to adjust the pelvis of the patient to a normal position;

in a case that the pelvis is at the normal position, a scoliosis treatment is configured to be performed on the patient based on this case;

the multi-degree-of-freedom seat unit is lifted or lowered when the at least three seat-adjustment support electric cylinders synchronously move upwards or downwards; and

the multi-degree-of-freedom seat unit is adjusted to be inclined by multiple inclination angles when the at least three seat-adjustment support electric cylinders move upwards or downwards asynchronously.

Preferably, the bearing-frame and hand-guard mechanism includes a system support frame and a hand guard bracket,

the system support frame provides a support frame for all electromechanical pneumatic and hydraulic components of a system, and a clinician is allowed to monitor a scoliosis parameter including a Cobb angle of the patient at a back of the patient; and

the patient is allowed to put hands on the hand guard bracket during the treatment process, and a position where the hand guard bracket is mounted includes a top of the robotic arm of the robot, a hand support position on a vertical column of a gantry of the robot, and another hand support position on a cross beam of the gantry of the robot.

Preferably, the dual-touch-screen display mechanism includes a medical-operation touch-screen display unit, an emergency-stop-button set safety protection unit, and a patient-observation touch-screen display unit,

the medical-operation touch-screen display unit is used for a medical operator to observe the scoliosis rehabilitation treatment process, and perform an operation through a doctor touch screen during the treatment process;

the emergency-stop-button set safety protection unit is configured to stop the rehabilitation robot system in an emergency and ensure safety; and

the patient-observation touch-screen display unit is configured to display a corrective rehabilitation process through a patient display screen, and the patient is allowed to observe the treatment process and the treatment outcome, and perform a medical requirement operation through a touch screen in the treatment process.

Compared with the conventional technology, the present application has at least the following beneficial effects.

Medical image data being read, body status data being measured and a real-time treatment status of the patient are uploaded to the cloud, and appropriate parameters for the treatment scheme are calculated by big data analytics and are transmitted back to the local. The scoliosis rehabilitation robot can provide precise corrective forces over three dimensions. With the scoliosis rehabilitation robot, the pelvis can be clamped and move upwards and downwards, and the seat can extend into a correction bed and be inclined by multiple angles, such that the force corrective treatment can be performed on the patient in various orientations. During the treatment process, the patient can know the treatment process and outcomes through real-time feedback. Relevant results obtained with technologies such as robotics, big data, cloud computing and the like can be used as a reference for subsequent treatments and treatments of other patients, and can be taken as a guide for a treatment prescription of home treatment devices and for design of a brace being daily worn.

In the solutions according to the present application, the scoliosis rehabilitation robot is competent for treating both C-shaped scoliosis and S-shaped scoliosis. The force application point can reach any part of the trunk of the patient, and the push force can be applied up and down along the vertical axis or around the vertical axis, and provide the three-dimensional push force to the patient, thereby realizing the multi-degree-of-freedom automated force treatment in the three-dimensional space to correct the lateral curvature and the vertebral rotation of scoliosis in a targeted manner. Moreover, the seat can be inclined by a certain angle towards any direction, so that the pelvis of the patient is adjusted to the normal position, thereby providing a more precise scoliosis treatment to the patient. Any inclination angle of the seat can be adjusted, and the seat can also be moved out of the treatment position. The force corrective treatment can be performed on the patient in various directions, where the force corrective treatment in various directions includes force corrective treatments in the sitting posture, in the standing posture and in the reclining posture. The hand guard is further provided in the scoliosis rehabilitation robot for the patient to put hands to alleviate fatigue for keeping receiving treatment during the required time period in the treatment process. During the treatment process of the patient, the back of the patient can be normally exposed, and correspondingly, the clinician monitoring the Cobb angle and other scoliosis parameters from the back of the patient may not be affected by the push arm, the seat, the display screen, the hand guard and other devices. The head traction device is further provided in the scoliosis rehabilitation robot to eliminate a pressure applied on the spine by a weight of the head, so that the treatment is more effective. A position of the robotic arm, a magnitude and the direction of the force, a magnitude of a clamping force of the clamping board of the seat, and the inclination angle of the seat of the scoliosis rehabilitation robot can be set through a control interface, and automatically achieve a preset effect in a way making the patient feel comfortable without excessive manual adjustments after being confirmed by the operator. Forces are provided at at least four points at the same time, which meets treatment demands of both C-shaped and S-shaped scoliosis.

The patient is provided with an automatic three-dimensional force application solution with multiple degrees of freedom. That is, the force can be applied up and down along the vertical axis or around the vertical axis. In the treatment of scoliosis, the seat is provided to fix the pelvis of the patient at the normal anatomical position. During the treatment process, the back of the patient can be exposed for the clinician to measure the degree of scoliosis with an imaging device. The patient is provided with a relatively large space in the treatment device to better cooperate with the treatment. During the treatment process, the magnitude and direction of the force can be set through the program, and an automatic corrective force treatment can be performed without the clinician manually operating the robotic arm. During the treatment process, the patient is provided with the display screen, which displays a treatment step being performed and an image of the back of the patient, so that the patient can know a shape after correction and have more confidence for the treatment. Configurations of various parameters of the correction results may be saved locally or uploaded to the cloud to provide data support for subsequent design and optimization of the brace of the patient. Multiple treatment methods are provided, including treatments in the sitting posture, the standing posture, and the reclining posture, according to the scoliosis status of the patient. At least one camera is provided to capture or record the image of the back of the patient during the treatment in real time, so that the patient can know about the treatment step being performed. Relevant setting parameters of the scoliosis rehabilitation robot and relevant information of the patient may be saved locally or uploaded to the cloud, to provide a reference and a guide for the treatment of the patient and other patients by the scoliosis rehabilitation robot, subsequent home treatments, bracing design, and adjustments of the brace of the patient.

The intelligent scoliosis rehabilitation robot with the cloud storage function is described in the present application. Other advantages, purposes and features of the present application may be partially embodied through the following description, and may be partially understood by those skilled in the art through studying and practice of the present application.

## BRIEF DESCRIPTION OF THE DRAWINGS

The accompanying drawings are intended to provide a further understanding of the present application and constitute a part of the specification. Together with the embodiments of the present application, the accompanying drawings are used to explain the present application, and do not constitute a limitation to the present application.

FIG. 1 is a block diagram of a system of an intelligent scoliosis rehabilitation robot with a cloud storage function;

FIG. 2 is a first view of the intelligent scoliosis rehabilitation robot with the cloud storage function according to an embodiment of the present application;

FIG. 3 is a second view of the intelligent scoliosis rehabilitation robot with the cloud storage function according to the embodiment of the present application; and

FIG. 4 is a view of a multi-degree-of-freedom seat unit of the intelligent scoliosis rehabilitation robot with the cloud storage function according to an embodiment of the present application.

## DETAILED DESCRIPTION OF THE EMBODIMENTS

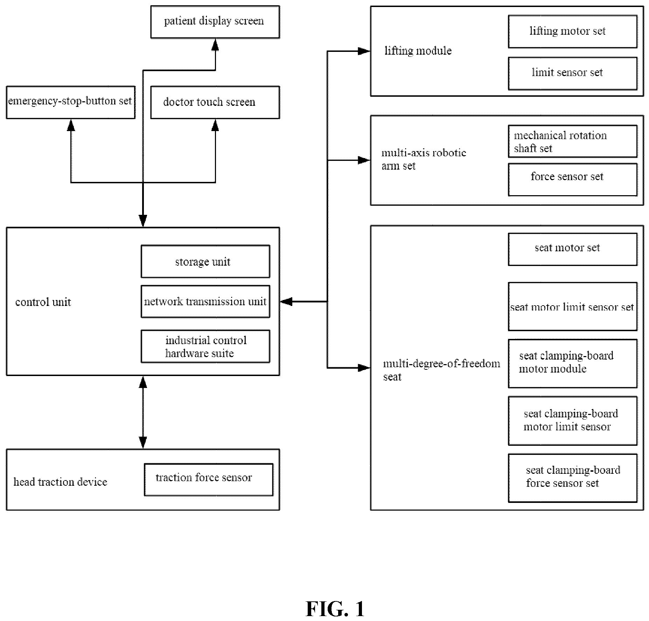

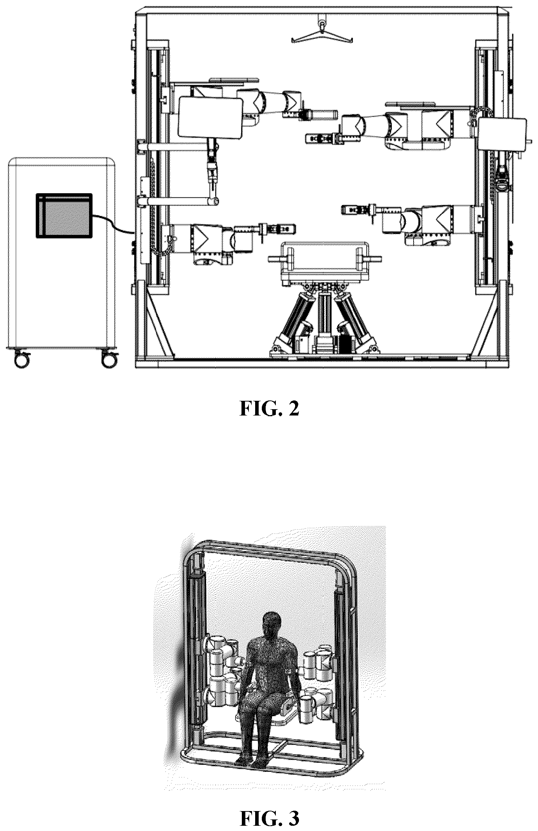

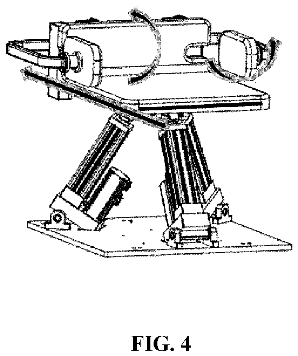

The present application is further described in detail hereinafter in conjunction with the accompanying drawings and embodiments, so that the present application can be implemented by those skilled in the art based on the content of the specification. As shown in FIG. 1 to FIG. 4, an intelligent scoliosis rehabilitation robot with a cloud storage function is provided according to the present application, including a head-support electroencephalogram and near-infrared monitoring module, a scoliosis body shape status measurement module, a individualized-parameter cloud analysis module, and a program-control and robotics actuator module.

The head-support electroencephalogram and near-infrared monitoring module is configured to support a head of a patient during a spinal correction process, monitor an electroencephalogram, a pulse rate, oxygen saturation, a fast optical neuronal signal, and a brain physiological signal of the patient, and adjust a corrective force.

The scoliosis body shape status measurement module is configured to measure a scoliosis status and a body shape status of the patient.

The individualized-parameter cloud analysis module is configured to transmit scoliosis body shape status measurement result and medical examination data to a cloud storage analysis system, and automatically generate an individualized adaptive parameter for the patient.

The program-control and robotics actuator module is configured to import the individualized adaptive parameter into a scoliosis rehabilitation control program, and control an actuator mechanism of a rehabilitation robot to perform intelligent individualized rehabilitation correction on the patient.

The operation principle of the above technical solution is as follows. The intelligent scoliosis rehabilitation robot with the cloud storage function is provided according to the present application, including the head-support electroencephalogram and near-infrared monitoring module, the scoliosis body shape status measurement module, the individualized-parameter cloud analysis module, and the program-control and robotics actuator module. The head-support electroencephalogram and near-infrared monitoring module is configured to support the head of the patient during the spinal correction process, monitor the electroencephalogram, the pulse rate, the oxygen saturation, the fast optical neuronal signal, and the brain physiological signal of the patient, and adjust the corrective force. The scoliosis body shape status measurement module is configured to measure the scoliosis status and the body shape status of the patient. The individualized-parameter cloud analysis module is configured to transmit the scoliosis body shape status measurement result and the medical examination data to the cloud storage analysis system, and automatically generate the individualized adaptive parameter for the patient. The program-control and robotics actuator module is configured to import the individualized adaptive parameter into the scoliosis rehabilitation control program, and control the actuator mechanism of the rehabilitation robot to perform the individualized intelligent rehabilitation correction on the patient. In this solution, a correction mechanism of the scoliosis rehabilitation robot has at least two lifting modules and at least four robotic arms, and is competent for treating both C-shaped scoliosis and S-shaped scoliosis. Each robotic arm can move upwards and downwards along the at least two lifting modules, and has at least three rotation axes, so that a force application point can be set at any part of a trunk of the patient, and a push force can be applied up and down along the vertical axis or around the vertical axis, so as to provide a three-dimensional push force to the patient, thereby realizing multi-degree-of-freedom automated force treatment in a three-dimensional space. Moreover, the scoliosis rehabilitation robot is further provided with a seat, and a clamping board is provided on the seat to automatically fix a pelvis of the patient. The seat is supported by three electric cylinders, and may be vertically moved or inclined with one or more degrees of freedom, so that the seat can be inclined by a certain angle in any direction, and the treatment of scoliosis is provided to the patient after the pelvis of the patient is moved to a normal position. The seat is lifted or lowered when the three electric cylinders synchronously move upwards or downwards, and the seat is adjusted to be inclined by any inclination angle when the three electric cylinders move upwards or downwards asynchronously. The seat can also be removed to facilitate the force corrective treatment of the patient in various postures such as in a standing posture. A hand guard is further provided in the scoliosis rehabilitation robot for the patient to put hands. During the treatment of the patient, a back of the patient can be normally exposed, and correspondingly, a clinician monitoring the Cobb angle and other scoliosis parameters on the back of the patient through a scoliosis assessment device may not be affected by the push arm, the seat, a display screen, the hand guard and other devices. A head traction device is further provided in the scoliosis rehabilitation robot to eliminate a pressure applied on the spine by a weight of the head. A position of the robotic arm, a magnitude and the direction of the force, a magnitude of a clamping force of the clamping board of the seat, and the inclination angle of the seat of the scoliosis rehabilitation robot can be set through a control interface, and automatically achieve a preset effect in a way making the patient feel comfortable after being confirmed by an operator.

The beneficial effect of the above technical solution is as follows. The intelligent scoliosis rehabilitation robot with the cloud storage function according to the present application includes the head-support electroencephalogram and near-infrared monitoring module, the scoliosis body shape status measurement module, the individualized-parameter cloud analysis module, and the program-control and robotics actuator module. The head-support electroencephalogram and near-infrared monitoring module is configured to support the head of the patient during the spinal correction process, monitor the electroencephalogram, the pulse rate, the oxygen saturation, the fast optical neuronal signal, and the brain physiological signal of the patient, and adjust the corrective force or indicate a comfort level during the treatment process. The scoliosis body shape status measurement module is configured to measure the scoliosis status and the body shape status of the patient. The individualized-parameter cloud analysis module is configured to transmit the scoliosis body shape status measurement result and the medical examination data to the cloud storage analysis system, and automatically generate the individualized adaptive parameter of the patient. The program-control and robotics actuator module is configured to import the individualized adaptive parameter into the scoliosis rehabilitation control program, and control the actuator mechanism of the rehabilitation robot to perform the individualized intelligent rehabilitation correction on the patient. In this solution, the correction mechanism of the scoliosis rehabilitation robot has at least two lifting modules and at least four robotic arms, and is competent for treating both C-shaped scoliosis and S-shaped scoliosis. Each robotic arm can move upwards and downwards along the at least two lifting modules, and has at least three rotation axes, so that a force application point can be set at any part of the trunk of the patient, and the push force can be applied up and down along the vertical axis or around the vertical axis, so as to provide the three-dimensional push force to the patient, thereby realizing the multi-degree-of-freedom automated force treatment in the three-dimensional space.

Moreover, the scoliosis rehabilitation robot is further provided with the seat, and the clamping board is provided on the seat to automatically fix the pelvis of the patient. The seat is supported by the three electric cylinders, and may be vertically moved or inclined with one or more degrees of freedom, so that the seat can be inclined by a certain angle in any direction, and the treatment of scoliosis is provided to the patient after the pelvis of the patient is adjusted to the normal position. The seat can be lifted or lowered when the three electric cylinders synchronously move upwards or downwards, and the seat can be adjusted to be inclined by any inclination angle when the three electric cylinders move upwards or downwards asynchronously.

The seat can also be removed to facilitate the force corrective treatment of the patient in various postures such as in a standing posture. The hand guard is further provided in the scoliosis rehabilitation robot for the patient to put hands, so as to alleviate fatigue for keeping receiving treatment during a required time period in the treatment process. During the treatment, the back of the patient can be normally exposed, and correspondingly, the clinician monitoring the Cobb angle and other scoliosis parameters on the back of the patient may not be affected by the push arm, the seat, the display screen, the hand guard and other devices. The head traction device is further provided in the scoliosis rehabilitation robot to eliminate the pressure applied on the spine by the weight of the head, so that the treatment is more effective. The position of the robotic arm, the magnitude and the direction of the force, the magnitude of the clamping force of the clamping board of the seat, and the inclination angle of the seat of the scoliosis rehabilitation robot can be set through the control interface, and automatically achieve the preset effect in the way making the patient feel comfortable without excessive manual adjustments after being confirmed by the operator. Forces are provided at at least four points at the same time, which meets treatment demands of both C-shaped and S-shaped scoliosis.

The patient is provided with an automatic three-dimensional force application solution with multiple degrees of freedom. That is, the force can be applied up and down along the vertical axis or around the vertical axis. In the treatment of scoliosis, the seat is provided to fix the pelvis of the patient at the normal anatomical position. During the treatment process, the back of the patient can be exposed for the clinician to measure the degree of scoliosis by an imaging device. The patient is provided with a relatively large space to better coordinate the treatment. During the treatment process, the magnitude and the direction of the force can be set through the program, and an automatic corrective force treatment can be performed without the clinician manually operating the robotic arm. During the treatment process, the patient is provided with a display screen, which can display a treatment step being performed and an image of the back of the patient, so that the patient can know a shape after correction and have more confidence for the treatment. Configurations of parameters of the device during the treatment process, especially when the correction has relatively good results, may be saved locally or uploaded to the cloud for subsequent design of the brace of the patient. Multiple treatment methods are provided, including treatments in the sitting posture, the standing posture, and the reclining posture, according to the scoliosis status of the patient. The scoliosis rehabilitation robot is provided with a camera, and the image of the back of the patient during the treatment can be displayed on a patient display screen in real time, so that the patient can know about the treatment step being performed. Relevant setting parameters of the scoliosis rehabilitation robot and relevant information of the patient may be saved locally or uploaded to the cloud, so as to provide a reference and a guide for the treatment of the patient and other patients by the scoliosis rehabilitation robot, subsequent home treatments, and, design and adjustments of the brace of the patient.

In an embodiment, the head-support electroencephalogram and near-infrared monitoring module includes a head-support suspended lifting sub-module, an electroencephalogram and near-infrared signal acquisition sensor sub-module, and an electrode and near-infrared optical sensor connection monitoring and tracking sub-module.

The head-support suspended lifting sub-module is configured to support the head of the patient through a head traction device during the spinal correction process, and acquire traction force data through a traction force sensor of the head traction device.

The electroencephalogram and near-infrared signal acquisition sensor sub-module is configured to acquire the electroencephalogram, the pulse rate, the oxygen saturation, the fast optical neuronal signal and the brain physiological signal of the patient through a signal acquisition electrode and a near-infrared optical sensor.

The electrode and near-infrared optical sensor connection monitoring and tracking sub-module is configured to assist to analyze whether a treatment process is proper according to the physiological signals including the electroencephalogram, the pulse rate, the oxygen saturation, and the fast optical neuronal signal of the patient, and monitor an emergency where the patient is not able to call for help, such that a doctor can monitor a status of the patient through the physiological signals including the electroencephalogram, the pulse rate, the oxygen saturation, and the fast optical neuronal signal, and further adjusts the corrective force.

The operation principle of the above technical solution is based on mechanical traction, lifting, and support, and the electroencephalogram and near-infrared signal acquisition, sensing and monitoring. The head-support electroencephalogram and near-infrared monitoring module includes the head-support suspended lifting sub-module, the electroencephalogram and near-infrared signal acquisition sensor sub-module, and the electrode and near-infrared optical sensor connection monitoring and tracking sub-module. The head-support suspended lifting sub-module is configured to support the head of the patient through the head traction device during the spinal correction process, and acquire the traction force data through the traction force sensor of the head traction device. The electroencephalogram and near-infrared signal acquisition sensor sub-module is configured to acquire the electroencephalogram, the pulse rate, the oxygen saturation, and the fast optical neuronal signal of the patient through the signal acquisition sensor. The electrode and near-infrared optical sensor connection monitoring and tracking sub-module is configured to assist to analyze whether the treatment process is proper according to the electroencephalogram, the pulse rate, the oxygen saturation, the fast optical neuronal signal and the brain physiological signal of the patient, monitor the emergency where the patient cannot call for help, such that the doctor can monitor the status of the patient through the electroencephalogram, the pulse rate, the oxygen saturation, the fast optical neuronal signal and the brain physiological signal, and further adjust the corrective force.

The beneficial effect of the above technical solution is as follows. The head-support electroencephalogram and near-infrared monitoring module includes the head-support suspended lifting sub-module, the electroencephalogram and near-infrared signal acquisition sensor sub-module, and the electroencephalogram-electrode and near-infrared optical sensor connection monitoring and tracking sub-module. The head-support suspended lifting sub-module is configured to support the head of the patient through the head traction device during the spinal correction process, and acquire traction data through the traction force sensor of the head traction device. The electroencephalogram and near-infrared signal acquisition sensor sub-module is configured to acquire the electroencephalogram, the pulse rate, the oxygen saturation, the fast optical neuronal signal and the brain physiological signal of the patient through the signal acquisition electrode. The electroencephalogram-electrode and near-infrared optical sensor connection monitoring and tracking sub-module is configured to assist to analyze whether the treatment process is proper according to the electroencephalogram, the pulse rate, the oxygen saturation, the fast optical neuronal signal and the brain physiological signal of the patient, monitor the emergency where the patient cannot call for help, such that the doctor can monitor the status of the patient through the electroencephalogram, the pulse rate, the oxygen saturation, the fast optical neuronal signal and the brain physiological signal, and further adjust the corrective force.

In an embodiment, the scoliosis body shape status measurement module includes a scoliosis status measurement sub-module, a patient body shape status measurement sub-module, and a rehabilitation status monitoring and measurement sub-module.

The scoliosis status measurement sub-module is configured to read a scoliosis medical image, and assist a doctor to measure the scoliosis status.

The patient body shape status measurement sub-module is configured to measure the body shape status of the patient, and measure a height, a weight, a body mass index, a subcutaneous fat thickness, and body measurements data.

The rehabilitation status monitoring and measurement sub-module is configured to monitor a treatment status of the patient and measure a treatment outcome.

The operation principle of the above technical solution is based on the monitoring and measurements of the scoliosis status, the body shape status of the patient and a rehabilitation status. The scoliosis body shape status measurement module includes the scoliosis status measurement sub-module, the patient body shape status measurement sub-module, and the rehabilitation status monitoring and measurement sub-module. The scoliosis status measurement sub-module is configured to read the scoliosis medical image data, and assist the doctor to measure the scoliosis status. The patient body shape status measurement sub-module is configured to measure the body shape status of the patient, and measure the height, the weight, the body mass index, the subcutaneous fat thickness, and the body measurements data. The rehabilitation status monitoring and measurement sub-module is configured to monitor the treatment status of the patient and measure the treatment outcome.

The beneficial effect of the above technical solution is as follows. The scoliosis body shape status measurement module includes the scoliosis status measurement sub-module, the patient body shape status measurement sub-module, and the rehabilitation status monitoring and measurement sub-module. The scoliosis status measurement sub-module is configured to read the scoliosis medical image data, and assist the doctor to measure the scoliosis status. The patient body shape status measurement sub-module is configured to measure the body shape status of the patient, and measure the height, the weight, the body mass index, the subcutaneous fat thickness, and the body measurements data. The rehabilitation status monitoring and measurement sub-module is configured to monitor the treatment status of the patient and measure the treatment outcome.

In an embodiment, the individualized-parameter cloud analysis module includes a cloud-platform and data-transmission sub-module, a medical examination data storage sub-module, and a cloud parameter analysis and storage sub-module.

The cloud-platform and data-transmission sub-module is configured to upload scoliosis body shape status measurement data to a cloud, so as to provide a reference and a guide for a treatment of the patient and other patients by the scoliosis rehabilitation robot, and for a subsequent home treatment of the patient, and bracing design and adjustment.

The medical examination data storage sub-module is configured to transmit medical examination data, and store scoliosis body shape status measurement data and the medical examination data to the cloud.

The cloud parameter analysis and storage sub-module is configured to perform cloud analysis on the scoliosis body shape status measurement data and the medical examination data, and automatically generate the individualized adaptive parameter of the patient.

The operation principle of the above technical solution is as follows. The scoliosis body shape status measurement data is processed at the cloud with cloud data transmission, cloud storage and cloud analysis. The individualized-parameter cloud analysis module includes the cloud-platform and data-transmission sub-module, the medical examination data storage sub-module, and the cloud parameter analysis and storage sub-module. The cloud-platform and data-transmission sub-module is configured to upload the scoliosis body shape status measurement data to the cloud, so as to provide a reference and a guide for the treatment of the patient and other patients by the scoliosis rehabilitation robot, and for the subsequent home treatment of the patient, and the bracing design and adjustment. The medical examination data storage sub-module is configured to transmit the medical examination data, and store the scoliosis body shape status measurement data and the medical examination data to the cloud. The cloud parameter analysis and storage sub-module is configured to perform cloud analysis on the scoliosis body shape status measurement data and the medical examination data, and automatically generate the individualized adaptive parameter of the patient.

The beneficial effect of the above technical solution is as follows. The individualized-parameter cloud analysis module includes the cloud-platform and data-transmission sub-module, the medical examination data storage sub-module, and the cloud parameter analysis and storage sub-module. The cloud-platform and data-transmission sub-module is configured to upload scoliosis body shape status measurement data to the cloud, so as to provide a reference and a guide for the treatment of the patient and other patients by the scoliosis rehabilitation robot, and for the subsequent home treatment of the patient, and the bracing design and adjustment. The medical examination data storage sub-module is configured to transmit the medical examination data, and store the scoliosis body shape status measurement data and the medical examination data to the cloud. The cloud parameter analysis and storage sub-module is configured to perform cloud analysis on the scoliosis body shape status measurement data and the medical examination data, and automatically generate the individualized adaptive parameter of the patient. The scoliosis rehabilitation robot is provided with a camera, and the image of the back of the patient during the treatment can be displayed on the patient display screen in real time, so that the patient can know about the treatment step being performed. Relevant setting parameters of the scoliosis rehabilitation robot and relevant information of the patient may be saved locally or uploaded to the cloud, so as to provide a reference and a guide for the treatment of the patient and other patients by the scoliosis rehabilitation robot, subsequent home treatments, and bracing design and adjustments of the patient.

In an embodiment, the program-control and robotics actuator module includes a cloud data import program sub-module, a scoliosis rehabilitation program control sub-module, and a control and robotics actuator sub-module.

The cloud data import program sub-module is configured to import the individualized adaptive parameter and cloud storage data into a control unit. The control unit includes a storage unit, a network transmission unit, and an industrial control hardware suite, and the individualized adaptive parameter and the cloud storage data can be imported into the control unit through the network transmission unit, and stored in a local storage unit.

The scoliosis rehabilitation program control sub-module is configured to read the individualized adaptive parameter in the local storage unit, input the individualized adaptive parameter into a scoliosis rehabilitation program in the industrial control hardware suite, and generate an individualized adaptive scoliosis rehabilitation program.

The control and robotics actuator sub-module is configured to control the scoliosis rehabilitation robot to perform a scoliosis rehabilitation treatment process through the individualized adaptive scoliosis rehabilitation program, start the scoliosis rehabilitation control program, and control an actuator mechanism of the scoliosis rehabilitation robot to perform the intelligent individualized rehabilitation correction on the patient.

The operation principle of the above technical solution is as follows. The individualized adaptive parameter and the cloud storage data are imported into a standard program of the control unit through network data transmission and the industrial control hardware suite, and the individualized adaptive scoliosis rehabilitation program is generated. The program-control and robotics actuator module includes the cloud data import program sub-module, the scoliosis rehabilitation program control sub-module, and the control and robotics actuator sub-module. The cloud data import program sub-module is configured to import the individualized adaptive parameter and the cloud storage data into the control unit. The control unit includes the storage unit, the network transmission unit, and the industrial control hardware suite, and the individualized adaptive parameter and the cloud storage data can be imported into the control unit through the network transmission unit, and stored in the local storage unit. The scoliosis rehabilitation program control sub-module is configured to read the individualized adaptive parameter in the local storage unit, input the individualized adaptive parameter into the scoliosis rehabilitation program in the industrial control hardware suite, and generate the individualized adaptive scoliosis rehabilitation program. The control and robotics actuator sub-module is configured to control the scoliosis rehabilitation robot to perform the scoliosis rehabilitation treatment process through the individualized adaptive scoliosis rehabilitation program, start the scoliosis rehabilitation control program, and control the actuator mechanism of the scoliosis rehabilitation robot to perform the intelligent individualized rehabilitation correction on the patient.

The beneficial effect of the above technical solution is as follows. The program-control and robotics actuator module includes the cloud data import program sub-module, the scoliosis rehabilitation program control sub-module, and the control and robotics actuator sub-module. The cloud data import program sub-module is configured to import the individualized adaptive parameter and the cloud storage data into the control unit. The control unit includes the storage unit, the network transmission unit, and the industrial control hardware suite, and the individualized adaptive parameter and the cloud storage data can be imported into the control unit through the network transmission unit, and stored in the local storage unit. The scoliosis rehabilitation program control sub-module is configured to read the individualized adaptive parameter in the local storage unit, input the individualized adaptive parameter into the scoliosis rehabilitation program in the industrial control hardware suite, and generate the individualized adaptive scoliosis rehabilitation program. The control and robotics actuator sub-module is configured to control the scoliosis rehabilitation robot to perform the scoliosis rehabilitation treatment process through the individualized adaptive scoliosis rehabilitation program, start the scoliosis rehabilitation control program, and control the actuator mechanism of the scoliosis rehabilitation robot to perform the intelligent individualized rehabilitation correction on the patient.

In an embodiment, the control and robotics actuator sub-module includes a scoliosis correction mechanism, a multi-angle pelvis fixation mechanism, a bearing-frame and hand-guard mechanism, and a dual- touch-screen display mechanism.

The scoliosis correction mechanism is configured to provide a three-dimensional push force for scoliosis correction of the patient to realize a multi-degree-of-freedom automated force treatment in a three-dimensional space.

The multi-angle pelvis fixation mechanism is configured to automatically fix a pelvis of the patient, adjust a position of the pelvis of the patient, and perform adjustments adjust in multiple inclination angles.

The bearing-frame and hand-guard mechanism is configured to serve as a hand guard during a scoliosis rehabilitation process, thus the patient puts hands onto the hand guard during the treatment process to alleviate fatigue for keeping receiving treatment during a required time period in the treatment process.

The dual-touch-screen display mechanism is configured for a touch screen operation and for displaying a status of the treatment process through the dual-touch-screen display mechanism facing both the patient and a medical staff.

The operation principle of the above technical solution is based on calculations of forces in multiple directions in the three-dimensional space and an equivalent stress. The control and robotics actuator sub-module includes the scoliosis correction mechanism, the multi-angle pelvis fixation mechanism, the bearing-frame and hand-guard mechanism, and the dual-touch-screen display mechanism. The scoliosis correction mechanism is configured to provide the three-dimensional push force for the scoliosis correction of the patient to realize the multi-degree-of-freedom automated force treatment in the three-dimensional space. The multi-angle pelvis fixation mechanism is configured to automatically fix the pelvis of the patient, adjust the position of the pelvis of the patient, and perform adjustments in multiple inclination angles. The bearing-frame and hand-guard mechanism is configured to serve as the hand guard during the scoliosis rehabilitation process, such that the patient puts hands onto the hand guard during the treatment process to alleviate fatigue for keeping receiving treatment during the required time period in the treatment process.

The dual-touch-screen display mechanism is configured for the touch screen operation and for displaying the status of the treatment process through the dual-touch-screen display mechanism facing both the patient and the medical staff. The equivalent stress of a force applied on the spine in the scoliosis correction is calculated by the following equation.

$$Fvmn = \frac{1}{Stc} \sqrt{\frac{1}{2}[(Fvm1 - Fvm2)^2 + (Fvm2 - Fvm3)^2 + (Fvm3 - Fvm1)^2]}$$

In the equation, Fvmn represents the equivalent stress of the force applied on the spine in the scoliosis correction. Stc represents an equivalent-stress adjustment coefficient, which relates to the degree of scoliosis, the body status, the body shape, bone density and muscle endurance of the patient, and is obtained based on medical analysis by the doctor in conjunction with scoliosis medical big data statistics in the cloud storage. Fvm1 represents a first correction principal stress of scoliosis, Fvm2 represents a second correction principal stress of scoliosis, and Fvm3 represents a third correction principal stress of scoliosis. According to the calculation of the equivalent stress of the force applied on the spine in the scoliosis correction, the multiple corrective forces during the correction process are expected to reach a minimum corrective force required by the correction of scoliosis, and should not exceed the equivalent stress that damages the spine.

The beneficial effect of the above technical solution is as follows. The control and robotics actuator sub-module includes the scoliosis correction mechanism, the multi-angle pelvis fixation mechanism, the bearing-frame and hand-guard mechanism, and the dual- touch-screen display mechanism. The scoliosis correction mechanism is configured to provide the three-dimensional push force for the scoliosis correction of the patient to realize the multi-degree-of-freedom automated force treatment in the three-dimensional space. The multi-angle pelvis fixation mechanism is configured to automatically fix the pelvis of the patient, adjust the position of the pelvis of the patient, and adjust in multiple inclination angles. The bearing-frame and hand-guard mechanism is configured to serve as the hand guard during the scoliosis rehabilitation process, such that the patient puts hands onto the hand guard during the treatment process to alleviate fatigue for keeping receiving treatment during the required time period in the treatment process. The dual-touch-screen display mechanism is configured for the touch screen operation and for displaying the status of the treatment process through the dual-touch-screen display mechanism facing both the patient and the medical staff. The equivalent stress of the force applied on the spine in the scoliosis correction is calculated. In the equation, Fvmn represents the equivalent stress of the force applied on the spine in the scoliosis correction. Stc represents the equivalent-stress adjustment coefficient, which relates to the degree of scoliosis, the body status, the body shape, the bone density and the muscle endurance of the patient, and is obtained based on the medical analysis by the doctor in conjunction with the scoliosis medical big data statistics in the cloud storage. Fvm1 represents the first scorrection principal stress of scoliosis, Fvm2 represents the second correction principal stress of scoliosis, and Fvm3 represents the third correction principal stress of scoliosis. According to the calculation of the equivalent stress of the force applied on the spine in the scoliosis correction, the multiple corrective forces during the correction process are expected to reach the minimum corrective force required by the correction of scoliosis, and should not exceed the equivalent stress that damages the spine, thereby increasing the precision of the corrective forces and improving the physical adaptability of the body of the patient.

In an embodiment, the scoliosis correction mechanism includes a lifting module unit, a multi-axis robotic arm set unit, a scoliosis correction arm, and an image acquisition module.

The lifting module unit is configured to drive a robotic arm of the scoliosis rehabilitation robot to move upwards and downwards, and the lifting module unit includes at least two lifting modules, and each of the at least two lifting modules includes a lifting motor set and a limit sensor set.

The multi-axis robotic arm set unit is configured to drive the scoliosis correction arm to move along a horizontal direction and push a scoliosis correction arm. The multi-axis robotic arm set unit includes at least four multi-axis robotic arm sets, and each of the at least four multi-axis robotic arm sets includes a mechanical rotation shaft set and a force sensor set. The mechanical rotation shaft set is connected and mounted to the at least two lifting modules, and the force sensor set includes multiple force sensors and is mounted at a connection position of a rotation pivot and a connecting rod of the mechanical rotation shaft set. The multi-axis robotic arm set has rotation axes along an X direction, a Y direction, and a Z direction over three dimensions in a space, and is configured to drive the scoliosis correction arm such that the scoliosis correction arm can apply a force at any position of a trunk of the patient, and a direction of the push force moves upwards and downwards along the vertical axis or rotates around the vertical axis, to provide the three-dimensional push force to the patient for correction to realize the multi-degree-of-freedom automated force treatment in the three-dimensional space. The multi-axis robotic arm set is configured to control the robotic arm to automatically move upwards and downwards along the vertical axis or rotate around the vertical axis and provide a multi-degree-of-freedom corrective force through a setting made by a medical stuff on an interface of the dual-touch-screen display mechanism in a medical operation.

The scoliosis correction arm is configured to be driven by the robotic arm to be located at a position of a correction treatment of the patient to perform the scoliosis correction.

The image acquisition module is configured to acquire images of the treatment process from multiple perspectives through an angle-adjustable camera.

The operation principle of the above technical solution is based on the traction and lifting, the pivot and the connecting rod with multiple degrees of freedom, and the image acquisition. The scoliosis correction mechanism includes the lifting module unit, the multi-axis robotic arm set unit, the scoliosis correction arm, and the image acquisition module. The lifting module unit is configured to drive the robotic arm of the scoliosis rehabilitation robot to move upwards and downwards. The lifting module unit includes the at least two lifting modules, and each of the at least two lifting modules includes the lifting motor set and the limit sensor set. The multi-axis robotic arm set unit is configured to drive the scoliosis correction arm to move along the horizontal direction and push the scoliosis correction arm. The multi-axis robotic arm set unit includes the at least four multi-axis robotic arm sets, and each of the at least four multi-axis robotic arm sets include the mechanical rotation shaft set and the force sensor set. The mechanical rotation shaft set is connected and mounted to the at least two lifting modules, and the force sensor set includes the multiple force sensors and is mounted at the connection position of the rotation pivot and the connecting rod of the mechanical rotation shaft set. The multi-axis robotic arm set unit has the rotation axes along the X direction, the Y direction, and the Z direction over three dimensions in the space, and is configured to drive the scoliosis correction arm such that the scoliosis correction arm can apply the force at any position of the trunk of the patient, and the push force can be applied up and down along the vertical axis or around the vertical axis, so as to provide the three-dimensional push force to the patient for the correction and realize the multi-degree-of-freedom automated force treatment in the three-dimensional space. The multi-axis robotic arm set is configured to control the robotic arm to automatically move upwards and downwards along the vertical axis or rotate around the vertical axis and provide the multi-degree-of-freedom corrective force through the setting made by the medical stuff on the interface of the dual-touch-screen display mechanism in the medical operation. The scoliosis correction arm is configured to be driven by the robotic arm to be located to the position of the correction treatment of the patient to perform the scoliosis correction. The image acquisition module is configured to acquire the images of the treatment process from multiple perspectives through the angle-adjustable camera.

The beneficial effect of the above technical solution is as follows. The scoliosis correction mechanism includes the lifting module unit, the multi-axis robotic arm set unit, the scoliosis correction arm, and the image acquisition module. The scoliosis correction mechanism includes the lifting module unit, the multi-axis robotic arm set unit, the scoliosis correction arm, and the image acquisition module. The lifting module unit is configured to drive the robotic arm of the scoliosis rehabilitation robot to move upwards and downwards. The lifting module unit includes the at least two lifting modules, and each of the at least two lifting modules includes the lifting motor set and the limit sensor set. The multi-axis robotic arm set unit is configured to drive the scoliosis correction arm to move along the horizontal direction and push the scoliosis correction arm. The multi-axis robotic arm set unit includes the at least four multi-axis robotic arm sets, and each of the at least four multi-axis robotic arm sets include the mechanical rotation shaft set and the force sensor set. The mechanical rotation shaft set is connected and mounted to the at least two lifting modules, and the force sensor set includes the multiple force sensors and is mounted at the connection position of the rotation pivot and the connecting rod of the mechanical rotation shaft set. The multi-axis robotic arm set unit has the rotation axes along the X direction, the Y direction, and the Z direction over three dimensions in the space, and is configured to drive the scoliosis correction arm such that the scoliosis correction arm can apply the force at any position of the trunk of the patient, and the push force can be applied up and down along the vertical axis or around the vertical axis, so as to provide the three-dimensional push force to the patient for the correction to realize the multi-degree-of-freedom automated force treatment in the three-dimensional space and ensure the push forces to be safe and effective. The multi-axis robotic arm set is configured to control the robotic arm to automatically move upwards and downwards along the vertical axis or rotate around the vertical axis and provide the multi-degree-of-freedom corrective force through the setting made by the medical stuff on the interface of the dual-touch-screen display mechanism in the medical operation. The scoliosis correction arm is configured to be driven by the robotic arm to be located to the position of the correction treatment of the patient to perform the scoliosis correction. The image acquisition module is configured to acquire the images of the treatment process from multiple perspectives through the angle-adjustable camera. The patient is provided with the automatic three-dimensional force application solution with multiple degrees of freedom. That is, the direction of the force can move upwards and downwards along the vertical axis or rotate around the vertical axis. In the treatment of scoliosis, the seat is provided to fix the pelvis of the patient at the normal anatomical position. During the treatment process, the back of the patient can be exposed for the clinician to measure the degree of scoliosis with the imaging device. The patient is provided with a relatively large space to better cooperate with the treatment. During the treatment process, the magnitude and the direction of the force can be set through the program, and the automatic corrective force treatment can be performed without the clinician manually operating the robotic arm. During the treatment process, the patient is provided with the display screen, which displays the treatment step being performed and the image of the back of the patient, so that the patient can know the shape after the correction and have more confidence for the treatment.

In an embodiment, the multi-angle pelvis fixation mechanism includes a multi-angle pelvis fixation mechanism.

The multi-angle pelvis fixation mechanism is used for a multi-posture force corrective treatment of the patient. The multi-degree-of-freedom seat unit includes a seat motor set, a seat clamping-board motor module, a seat motor limit sensor set, a seat clamping-board motor limit sensor set, and a seat clamping-board force sensor set. The seat motor set includes a seat-adjustment drive motor and at least three seat-adjustment support electric cylinders, and the at least three seat-adjustment support electric cylinders are fixedly connected to a rotation shaft of the seat-adjustment drive motor through a bolt. The multi-posture force corrective treatment includes force corrective treatments in a sitting posture, a standing posture, and a reclining posture. A seat surface of the multi-degree-of freedom seat unit is composed of a pull-out multi-layer board. An outer board of the pull-out multi-layer board is connected to the at least three seat-adjustment support electric cylinders, and an inner board of the pull-out multi-layer board is connected to the outer board through a slide rail. In a case that the inner board of the pull-out multi-layer board is pulled out, the seat surface extends to form a bed-shaped board surface, and the bed-shaped board surface is used for the force corrective treatment of the patient in the reclining posture through an adjustment to an angle of the bed-shaped board surface. In a case that the multi-degree-of-freedom seat unit is removed from a treatment position, the corrective treatment of the patient in the standing posture is configured to be performed.

The multi-degree-of-freedom seat unit is provided with a pelvis fixation clamping board that is configured to be driven by the seat clamping-board motor module to fix the pelvis of the patient.

The at least three seat-adjustment support electric cylinders are configured to support the multi-degree-of-freedom seat unit to make the multi-degree-of-freedom seat unit move upwards and downwards or be inclined with one or multiple degrees of freedom, such that the multi-degree-of-freedom seat unit is inclined by an angle towards any direction to adjust the pelvis of the patient to a normal position. In a case that the pelvis is at the normal position, a scoliosis treatment is configured to be performed on the patient based on this case. The multi-degree-of-freedom seat unit is lifted or lowered when the at least three seat-adjustment support electric cylinders synchronously move upwards or downwards. The multi-degree-of-freedom seat unit is adjusted to be inclined by multiple inclination angles when the at least three seat-adjustment support electric cylinders move upwards or downwards asynchronously

The operation principle of the above technical solution is based on multi-axis motor sets, the pull-out multi-layer board, and a triangular pivot for keeping stable. The multi-angle pelvis fixation mechanism includes the multi-degree-of-freedom seat unit that is used for the multi-posture force corrective treatment of the patient. The multi-degree-of-freedom seat unit includes the seat motor set, the seat clamping-board motor module, the seat motor limit sensor set, the seat clamping-board motor limit sensor set, and the seat clamping-board force sensor set. The seat motor set includes the seat-adjustment drive motor and the at least three seat-adjustment support electric cylinders, and the at least three seat-adjustment support electric cylinders are fixedly connected to the rotation shaft of the seat-adjustment drive motor through the bolt. The multi-posture force corrective treatment includes the force corrective treatments in the sitting posture, the standing posture, and the reclining posture. The seat surface of the multi-degree-of-freedom seat unit is composed of the pull-out multi-layer board. The outer board of the pull-out multi-layer board is connected to the at least three seat-adjustment support electric cylinders, and the inner board of the pull-out multi-layer board is connected to the outer board through the slide rail. In a case that the inner board of the pull-out multi-layer board is pulled out, the seat surface extends to form the bed-shaped board surface, and the bed-shaped board surface is used for the force corrective treatment of the patient in the reclining posture through the adjustment to the angle of the bed-shaped board surface. In a case that the multi-degree-of-freedom seat unit is removed from the treatment position, the corrective treatment of the patient in the standing posture is performed. The multi-degree-of-freedom seat unit is provided with the pelvis fixation clamping board that is configured to be driven by the seat clamping-board motor module to fix the pelvis of the patient. The at least three seat-adjustment support electric cylinders are configured to support the multi-degree-of-freedom seat unit to make the multi-degree-of-freedom seat unit move upwards and downwards or be inclined with one or multiple degrees of freedom, such that the multi-degree-of-freedom seat unit is inclined by an angle towards any direction to adjust the pelvis of the patient to the normal position. In a case that the pelvis is at the normal position, the scoliosis treatment is performed on the patient based on this case. The multi-degree-of-freedom seat unit is lifted or lowered when the at least three seat-adjustment support electric cylinders synchronously move upwards or downwards. The multi-degree-of-freedom seat unit is adjusted to be inclined by multiple inclination angles when the at least three seat-adjustment support electric cylinders move upwards or downwards asynchronously .

The beneficial effect of the above technical solution is as follows. The multi-angle pelvis fixation mechanism includes the multi-degree-of-freedom seat unit that is used for the multi-posture force corrective treatment of the patient. The multi-degree-of-freedom seat unit includes the seat motor set, the seat clamping-board motor module, the seat motor limit sensor set, the seat clamping-board motor limit sensor set, and the seat clamping-board force sensor set. The seat motor set includes the seat-adjustment drive motor and the at least three seat-adjustment support electric cylinders, and the at least three seat-adjustment support electric cylinders are fixedly connected to the rotation shaft of the seat-adjustment drive motor through the bolt. The multi-posture force corrective treatment includes force corrective treatments in the sitting posture, the standing posture, and the reclining posture. The seat surface of the multi-degree-of-freedom seat unit is composed of the pull-out multi-layer board. The outer board of the pull-out multi-layer board is connected to the at least three seat-adjustment support electric cylinders, and the inner board of the pull-out multi-layer board is connected to the outer board through the slide rail. In a case that the inner board of the pull-out multi-layer board is pulled out, the seat surface extends to form the bed-shaped board surface, and the bed-shaped board surface is used for the force corrective treatment of the patient in the reclining posture through the adjustment to the angle of the bed-shaped board surface. In a case that the multi-degree-of-freedom seat unit is removed from the treatment position, the corrective treatment of the patient in the standing posture is performed. The multi-degree-of-freedom seat unit is provided with the pelvis fixation clamping board that is configured to be driven by the seat clamping-board motor module to fix the pelvis of the patient. The at least three seat-adjustment support electric cylinders are configured to support the multi-degree-of-freedom seat unit to make the multi-degree-of-freedom seat unit move upwards and downwards or be inclined with one or multiple degrees of freedom, such that the multi-degree-of- freedom seat unit is inclined by an angle towards any direction to adjust the pelvis of the patient to the normal position. In a case that the pelvis is at the normal position, the scoliosis treatment is performed on the patient based on this case. The multi-degree-of-freedom seat unit is lifted or lowered when the at least three seat-adjustment support electric cylinders synchronously move upwards or downwards. The multi-degree-of-freedom seat unit is adjusted to be inclined by multiple inclination angles when the at least three seat-adjustment support electric cylinders move upwards or downwards asynchronously. During the treatment process, various treatments may be provided, including treatments in the sitting posture, the standing posture and the reclining posture, according to the scoliosis status of the patient.

In an embodiment, the bearing-frame and hand-guard mechanism includes a system support frame and a hand guard bracket.

The system support frame provides a support frame for all electromechanical pneumatic and hydraulic components of a system, and a clinician is allowed to monitor a scoliosis parameter including a Cobb angle of the patient at the back thereof.

The patient is allowed to put hands on the hand guard bracket during the treatment process, and a position where the hand guard bracket is mounted may be a top of the robotic arm of the robot, a hand support position on a vertical column of a gantry of the robot, and another hand support position on a cross beam of the gantry of the robot.

The operation principle of the above technical solution is as follows. A superior end vertebra and an inferior end vertebra of scoliosis are examined, which have the largest inclinations towards the concave side of scoliosis. An intervertebral space at the convex side of scoliosis is relatively large, and a first vertebra where an intervertebral space at the concave side starts to increase is considered not to belong to the scoliosis curvature. Therefore, one vertebra adjacent to the first vertebra is regarded as an end vertebra of the curvature. A cross line is drawn at an upper edge of the superior end vertebra, and another cross line is drawn at a lower edge of the inferior end vertebra. Two longitudinal lines respectively perpendicular to the two cross lines are drawn, and an angle between the two longitudinal lines is referred to as the Cobb angle. The bearing-frame and hand-guard mechanism includes the system support frame and the hand guard bracket. The system support frame provides the support frame for all electromechanical pneumatic and hydraulic components of the system, and the clinician is allowed to monitor the scoliosis parameter including the Cobb angle at the back of the patient. The patient is allowed to put hands on the hand guard bracket during the treatment process, and the position where the hand guard bracket is mounted may be the top of the robotic arm of the robot, the hand support position on the vertical column of the gantry of the robot, and the another hand support position on the cross beam of the gantry of the robot.

The beneficial effect of the above technical solution is as follows. The bearing-frame and hand-guard mechanism includes the system support frame and the hand guard bracket. The system support frame provides the support frame for all electromechanical pneumatic and hydraulic components of the system, and the clinician is allowed to monitor the scoliosis parameter including the Cobb angle at the back of the patient. The patient is allowed to put hands on the hand guard bracket during the treatment process, and the position where the hand guard bracket is mounted may be the top of the robotic arm of the robot, the hand support position on the vertical column of the gantry of the robot, and the another hand support position on the cross beam of the gantry of the robot. The support frame for all electromechanical pneumatic and hydraulic components of the system is provided, and various scoliosis parameters including the Cobb angle can be more comprehensively monitored. The patient can place hands during the treatment process, which makes the muscle groups of the body naturally relax during the treatment process, so as to prevent muscle fatigue during the treatment over a long time, thereby enhancing the treatment outcomes of the patient.

In an embodiment, the dual-touch-screen display mechanism includes a medical-operation touch-screen display unit, an emergency-stop-button set safety protection unit, and a patient-observation touch-screen display unit.

The medical-operation touch-screen display unit is for a medical operator to observe the scoliosis rehabilitation treatment process, and perform an operation through a doctor touch screen during the treatment process.

The emergency-stop-button set safety protection unit is configured to stop the rehabilitation robot system in an emergency and ensure safety.

The patient-observation touch-screen display unit is configured to display a corrective rehabilitation process through a patient display screen, and the patient is allowed to observe the treatment process and the treatment outcome, and perform a medical requirement operation through a touch screen in the treatment process.

The operation principle of the above technical solution is based on the dual touch screens for displaying and touch control, and the emergency-stop-button set for safety protection. The dual-touch-screen display mechanism includes the medical-operation touch-screen display unit, the emergency-stop-button set safety protection unit, and the patient-observation touch-screen display unit. The medical-operation touch-screen display unit is for the medical operator to observe the scoliosis rehabilitation treatment process, and perform an operation through the doctor touch screen during the treatment process. The emergency-stop-button set safety protection unit is configured to stop the rehabilitation robot system in the emergency and ensure safety. The patient-observation touch-screen display unit is configured to display the corrective rehabilitation process through the patient display screen, and the patient is allowed to observe the treatment process and the treatment outcome, and perform the medical requirement operation through the touch screen in the treatment process.

The beneficial effect of the above technical solution is as follows. The dual-touch-screen display mechanism includes the medical-operation touch-screen display unit, the emergency-stop-button set safety protection unit, and the patient-observation touch-screen display unit. The medical-operation touch-screen display unit is for the medical operator to observe the scoliosis rehabilitation treatment process, and perform an operation through the doctor touch screen during the treatment process. The emergency-stop-button set safety protection unit is configured to stop the rehabilitation robot system in the emergency and ensure safety. The patient-observation touch-screen display unit is configured to display the corrective rehabilitation process through the patient display screen, and the patient is allowed to observe the treatment process and the treatment outcome, and perform the medical requirement operation through the touch screen in the treatment process. The medical operation and the observation of the patient can communicate and interact with each other in time, and the safety of the system, the medical staff, and the patient can be protected comprehensively in time in the emergency.

Although the embodiments of the present application are disclosed hereinabove, the present application is not limited to the applications described in the specification and embodiments. The present application can be applied to various fields suitable for the present application. For those skilled in the art, additional modifications may be easily made. Therefore, without departing from the general concept defined by the claims and equivalent scope thereof, the present application is not limited to the specific details and the drawings illustrated and described herein.

## Claims

1. An intelligent scoliosis rehabilitation robot with a cloud storage function, comprising a head-support electroencephalogram and near-infrared monitoring module, a scoliosis body shape status measurement module, an individualized-parameter cloud analysis module, and a program-control and robotics actuator module, wherein

   the head-support electroencephalogram and near-infrared monitoring module is configured to support a head of a patient with scoliosis during a spinal correction process, monitor an electroencephalogram, a pulse rate, oxygen saturation, a fast optical neuronal signal, and a brain physiological signal of the patient, and adjust a corrective force;
   the scoliosis body shape status measurement module is configured to measure a scoliosis status and a body shape status of the patient;
   the individualized-parameter cloud analysis module is configured to transmit scoliosis body shape status measurement result and medical examination data to a cloud storage analysis system, and automatically generate an individualized adaptive parameter of the patient; and
   the program-control and robotics actuator module is configured to import an individualized adaptive parameter into a scoliosis rehabilitation control program, and control an actuator mechanism of the rehabilitation robot to perform intelligent individualized rehabilitation correction on the patient.

2. The intelligent scoliosis rehabilitation robot with the cloud storage function according to claim 1, wherein

the head-support electroencephalogram and near-infrared monitoring module comprises a head-support suspended lifting sub-module, an electroencephalogram and near-infrared signal acquisition sensor sub-module, and an electrode connection monitoring and tracking sub-module; wherein
the head-support suspended lifting sub-module is configured to support the head of the patient through a head traction device during the spinal correction process, and acquire traction force data through a traction force sensor of the head traction device;
the electroencephalogram and near-infrared signal acquisition sensor sub-module is configured to acquire the electroencephalogram, the pulse rate, the oxygen saturation, the fast optical neuronal signal, and the brain physiological signal of the patient through a signal acquisition electrode; and
the electrode connection monitoring and tracking sub-module is configured to assist to analyze whether a treatment process is proper according to the electroencephalogram, the pulse rate, the oxygen saturation, the fast optical neuronal signal and the brain physiological signal of the patient, and monitor an emergency where the patient fails to call for help, such that a doctor further adjusts the corrective force by monitoring a status of the patient through the electroencephalogram, the pulse rate, the oxygen saturation, the fast optical neuronal signal and the brain physiological signal.

3. The intelligent scoliosis rehabilitation robot with the cloud storage function according to claim 1, wherein

the scoliosis body shape status measurement module comprises a scoliosis status measurement sub-module, a patient body shape status measurement sub-module, and a rehabilitation status monitoring and measurement sub-module;
the scoliosis status measurement sub-module is configured to read a scoliosis medical image, and assist a doctor to measure the scoliosis status;
the patient body shape status measurement sub-module is configured to measure the body shape status of the patient, and measure a height, a weight, a body mass index, a subcutaneous fat thickness, and body measurements data; and
the rehabilitation status monitoring and measurement sub-module is configured to monitor a treatment status of the patient and measure a treatment outcome.

4. The intelligent scoliosis rehabilitation robot with the cloud storage function according to claim 1, wherein

the individualized-parameter cloud analysis module comprises a cloud-platform and data-transmission sub-module, a medical examination data storage sub-module, and a cloud parameter analysis and storage sub-module;
the cloud-platform and data-transmission sub-module is configured to upload scoliosis body shape status measurement data to a cloud, so as to provide a reference and a guide for a treatment of the patient and other patients by the scoliosis rehabilitation robot, and for a subsequent home treatment of the patient, and bracing design and adjustment;
the medical examination data storage sub-module is configured to transmit medical examination data, and store the scoliosis body shape status measurement data and the medical examination data to the cloud; and
the cloud parameter analysis and storage sub-module is configured to perform cloud analysis on the scoliosis body shape status measurement data and the medical examination data, and automatically generate the individualized adaptive parameter for the patient.

5. The intelligent scoliosis rehabilitation robot with the cloud storage function according to claim 1, wherein

the program-control and robotics actuator module comprises a cloud data import program sub-module, a scoliosis rehabilitation program control sub-module, and a control and robotics actuator sub-module; wherein
the cloud data import program sub-module is configured to import the individualized adaptive parameter and cloud storage data into a control unit, the control unit comprises a storage unit, a network transmission unit, and an industrial control hardware suite, and the individualized adaptive parameter and the cloud storage data are imported into the control unit through the network transmission unit, and stored in a local storage unit;
the scoliosis rehabilitation program control sub-module is configured to read the individualized adaptive parameter in the local storage unit, input the individualized adaptive parameter into a scoliosis rehabilitation program in the industrial control hardware suite, and generate an individualized adaptive scoliosis rehabilitation program; and

the control and robotics actuator sub-module is configured to control the scoliosis rehabilitation robot to perform a scoliosis rehabilitation treatment process through the individualized adaptive scoliosis rehabilitation program, start the scoliosis rehabilitation control program, and control the actuator mechanism of the scoliosis rehabilitation robot to perform the intelligent individualized rehabilitation correction on the patient.

6. The intelligent scoliosis rehabilitation robot with the cloud storage function according to claim 5, wherein

the control and robotics actuator sub-module comprises a scoliosis correction mechanism, a multi-angle pelvis fixation mechanism, a bearing-frame and hand-guard mechanism, and a dual-touch-screen display mechanism; the scoliosis correction mechanism is configured to provide a three-dimensional push force for scoliosis correction of the patient to realize a multi-degree-of-freedom automated force treatment in a three-dimensional space;

the multi-angle pelvis fixation mechanism is configured to automatically fix a pelvis of the patient, adjust a position of the pelvis of the patient, and perform adjustments in multiple inclination angles;

the bearing-frame and hand-guard mechanism is configured to serve as a hand guard during a scoliosis rehabilitation process, such that the patient puts hands onto the hand guard during a treatment process to alleviate fatigue for keeping receiving treatment during a required time period in the treatment process; and

the dual-touch-screen display mechanism is configured for a touch screen operation and for displaying a status of the treatment process through the dual-touch-screen display mechanism facing both the patient and a medical staff.

7. The intelligent scoliosis rehabilitation robot with the cloud storage function according to claim 6, wherein

the scoliosis correction mechanism comprises a lifting module unit, a multi-axis robotic arm set unit, a scoliosis correction arm, and an image acquisition module;

the lifting module unit is configured to drive a robotic arm of the scoliosis rehabilitation robot to move upwards and downwards, the lifting module unit comprises at least two lifting modules, and each of the at least two lifting modules comprises a lifting motor set and a limit sensor set;

the multi-axis robotic arm set unit is configured to drive the scoliosis correction arm to move along a horizontal direction and push the scoliosis correction arm, wherein

the multi-axis robotic arm set unit comprises at least four multi-axis robotic arm sets, and each of the at least four multi-axis robotic arm sets comprise a mechanical rotation shaft set and a force sensor set;

the mechanical rotation shaft set is connected and mounted to the at least two lifting modules, and the force sensor set comprises multiple force sensors and is mounted at a connection position of a rotation pivot and a connecting rod of the mechanical rotation shaft set;

the multi-axis robotic arm set has rotation axes along an X direction, a Y direction, and a Z direction over three dimensions in a space, and is configured to drive the scoliosis correction arm such that the scoliosis correction arm applies a force at any position of a trunk of the patient, and a push force is applied up and down along a vertical axis or around the vertical axis, so as to provide the three-dimensional push force to the patient for correction to realize the multi-degree-of-freedom automated force treatment in the three-dimensional space; and

the multi-axis robotic arm set is configured to control the robotic arm to automatically move upwards and downwards along the vertical axis or rotate around the vertical axis and provide a multi-degree-of-freedom corrective force through a setting made by a medical stuff on an interface of the dual-touch-screen display mechanism in a medical operation;

the scoliosis correction arm is configured to be driven by the robotic arm to be located at a position of a correction treatment of the patient to perform the scoliosis correction; and

the image acquisition module is configured to acquire images of the treatment process from multiple perspectives through an angle-adjustable camera.

8. The intelligent scoliosis rehabilitation robot with the cloud storage function according to claim 6, wherein

the multi-angle pelvis fixation mechanism comprises a multi-degree-of-freedom seat unit that is used for a multi-posture force corrective treatment of the patient;

the multi-degree-of-freedom seat unit comprises a seat motor set, a seat clamping-board motor module, a seat motor limit sensor set, a seat clamping-board motor limit sensor set, and a seat clamping-board force sensor set; the seat motor set comprises a seat-adjustment drive motor and at least three seat-adjustment support electric cylinders, and the at least three seat-adjustment support electric cylinders are fixedly connected to a rotation shaft of the seat-adjustment drive motor through a bolt;

the multi-posture force corrective treatment comprises force corrective treatments in a sitting posture, a standing posture, and a reclining posture;

a seat surface of the multi-degree-of-freedom seat unit is composed of a pull-out multi-layer board, an outer board of the pull-out multi-layer board is connected to the at least three seat-adjustment support electric cylinders, an inner board of the pull-out multi-layer board is connected to the outer board through a slide rail, and in a case that the inner board of the pull-out multi-layer board is pulled out, the seat surface extends to form a bed-shaped board surface, and the bed-shaped board surface is used for the force corrective treatment of the patient in the reclining posture through an adjustment to an angle of the bed-shaped board surface;

in a case that the multi-degree-of-freedom seat unit is removed from a treatment position, the corrective treatment of the patient in the standing posture is performed;

the multi-degree-of-freedom seat unit is provided with a pelvis fixation clamping board that is configured to be driven by the seat clamping-board motor module to fix the pelvis of the patient;

the at least three seat-adjustment support electric cylinders are configured to support the multi-degree-of-freedom seat unit to make the multi-degree-of-freedom seat unit move upwards and downwards or be inclined with one or multiple degrees of freedom, such that the multi-degree-of-freedom seat unit is inclined by an angle towards any direction to adjust the pelvis of the patient to a normal position;

in a case that the pelvis is at the normal position, a scoliosis treatment is performed on the patient based on this case;

the multi-degree-of-freedom seat unit is lifted or lowered when the at least three seat-adjustment support electric cylinders synchronously move upwards or downwards; and

the multi-degree-of-freedom seat unit is adjusted to be inclined by multiple inclination angles when the at least three seat-adjustment support electric cylinders move upwards or downwards asynchronously.

9. The intelligent scoliosis rehabilitation robot with the cloud storage function according to claim 6, wherein

the bearing-frame and hand-guard mechanism comprises a system support frame and a hand guard bracket; the system support frame provides a support frame for all electromechanical pneumatic and hydraulic components of a system, and a clinician is allowed to monitor a scoliosis parameter comprising a Cobb angle at a back of the patient; and

the patient is allowed to put hands on the hand guard bracket during the treatment process, and a position where the hand guard bracket is mounted is a top of the robotic arm of the robot, a hand support position on a vertical column of a gantry of the robot, and another hand support position on a cross beam of the gantry of the robot.

10. The intelligent scoliosis rehabilitation robot with the cloud storage function according to claim 6, wherein

the dual-touch-screen display mechanism comprises a medical-operation touch-screen display unit, an emergency-stop-button set safety protection unit, and a patient-observation touch-screen display unit; wherein the medical-operation touch-screen display unit is for a medical operator to observe the scoliosis rehabilitation treatment process, and perform an operation through a doctor touch screen during the treatment process;

the emergency-stop-button set safety protection unit is configured to stop the rehabilitation robot system in an emergency and ensure safety; and

the patient-observation touch-screen display unit is configured to display a corrective rehabilitation process through a patient display screen, and the patient is allowed to observe the treatment process and the treatment outcome, and perform a medical requirement operation through a touch screen in the treatment process.

**FIG. 1**

**FIG. 2**

**FIG. 3**

**FIG. 4**

<div style="text-align:center">**INTERNATIONAL SEARCH REPORT**</div>

| International application No. |
|---|
| **PCT/CN2023/090570** |

**A. CLASSIFICATION OF SUBJECT MATTER**

A61F5/01(2006.01)i; A61H1/02(2006.01)i; A61B5/369(2021.01)i; A61B5/0205(2006.01)i; A61B5/145(2006.01)i; A61B5/107(2006.01)i; G01G19/50(2006.01)i; A61B5/388(2021.01)i

According to International Patent Classification (IPC) or to both national classification and IPC

**B. FIELDS SEARCHED**

Minimum documentation searched (classification system followed by classification symbols)

IPC: A61F,A61B,A61H,G01G

Documentation searched other than minimum documentation to the extent that such documents are included in the fields searched

Electronic data base consulted during the international search (name of data base and, where practicable, search terms used)

CNABS, CNTXT, CNKI, VEN, WPABS, WPABSC, ENTXT, ENTXTC: 机械, 升, 降, 臂, 杆, 康复, 矫正, 脊, 椎, 参数, 值, 特定, 特异, mechanic+, lift, ris+, fall, arm?, bar?, correct+, spine?, vertibra, parameter?, value?, specific

**C. DOCUMENTS CONSIDERED TO BE RELEVANT**

| Category* | Citation of document, with indication, where appropriate, of the relevant passages | Relevant to claim No. |
|---|---|---|
| PX | CN 115054415 A (WUHAN KLAREDI MEDICAL TECHNOLOGY CO., LTD.) 16 September 2022 (2022-09-16)<br>claims 1-10 | 1-10 |
| X | CN 114028061 A (SHANGHAI QINGXUN INFORMATION TECHNOLOGY CO., LTD.) 11 February 2022 (2022-02-11)<br>description, paragraphs [0015]-[0031], and figures 1-3 | 1-6, 8-10 |
| A | CN 109172082 A (NANJING DRUM TOWER HOSPITAL) 11 January 2019 (2019-01-11)<br>entire document | 1-10 |
| A | CN 113974946 A (HARBIN QUANKE MEDICAL TECHNOLOGY DEVELOPMENT CO., LTD.) 28 January 2022 (2022-01-28)<br>entire document | 1-10 |
| A | CN 216136280 U (LI MING) 29 March 2022 (2022-03-29)<br>entire document | 1-10 |
| A | JP 2014087381 A (NAGANO GISHI KK) 15 May 2014 (2014-05-15)<br>entire document | 1-10 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**07 August 2023** | Date of mailing of the international search report<br><br>**07 August 2023** |
| Name and mailing address of the ISA/CN<br><br>**China National Intellectual Property Administration (ISA/CN)**<br>**China No. 6, Xitucheng Road, Jimenqiao, Haidian District, Beijing 100088** | Authorized officer<br><br><br><br>Telephone No. |

Form PCT/ISA/210 (second sheet) (July 2022)

**INTERNATIONAL SEARCH REPORT**

International application No.

**PCT/CN2023/090570**

| C. | DOCUMENTS CONSIDERED TO BE RELEVANT | |
|---|---|---|
| Category* | Citation of document, with indication, where appropriate, of the relevant passages | Relevant to claim No. |
| A | US 2010217166 A1 (SPINECORPORATION LTD.) 26 August 2010 (2010-08-26)<br>entire document | 1-10 |
| A | US 2016008206 A1 (DEVANABOYINA UDAYA SANKAR) 14 January 2016 (2016-01-14)<br>entire document | 1-10 |

Form PCT/ISA/210 (second sheet) (July 2022)

**INTERNATIONAL SEARCH REPORT**
Information on patent family members

International application No.

**PCT/CN2023/090570**

| Patent document cited in search report | | | Publication date (day/month/year) | Patent family member(s) | | | Publication date (day/month/year) |
|---|---|---|---|---|---|---|---|
| CN | 115054415 | A | 16 September 2022 | None | | | |
| CN | 114028061 | A | 11 February 2022 | None | | | |
| CN | 109172082 | A | 11 January 2019 | None | | | |
| CN | 113974946 | A | 28 January 2022 | None | | | |
| CN | 216136280 | U | 29 March 2022 | None | | | |
| JP | 2014087381 | A | 15 May 2014 | None | | | |
| US | 2010217166 | A1 | 26 August 2010 | CY | 1114392 | T1 | 31 August 2016 |
| | | | | CA | 2692174 | A1 | 24 August 2010 |
| | | | | CA | 2692174 | C | 24 November 2015 |
| | | | | AU | 2010200669 | A1 | 09 September 2010 |
| | | | | AU | 2010200669 | B2 | 03 April 2014 |
| | | | | PT | 2221028 | E | 29 August 2013 |
| | | | | JP | 2010194320 | A | 09 September 2010 |
| | | | | MY | 149429 | A | 30 August 2013 |
| | | | | DK | 2221028 | T3 | 29 July 2013 |
| | | | | PL | 2221028 | T3 | 31 January 2014 |
| | | | | EP | 2221028 | A1 | 25 August 2010 |
| | | | | EP | 2221028 | B1 | 05 June 2013 |
| | | | | US | 8795213 | B2 | 05 August 2014 |
| | | | | ES | 2425428 | T3 | 15 October 2013 |
| | | | | SG | 164354 | A1 | 29 September 2010 |
| | | | | GB | 0903093 | D0 | 08 April 2009 |
| | | | | GB | 2467974 | A | 25 August 2010 |
| | | | | GB | 2467974 | B | 05 January 2011 |
| | | | | KR | 20100097070 | A | 02 September 2010 |
| | | | | KR | 101532069 | B1 | 06 July 2015 |
| US | 2016008206 | A1 | 14 January 2016 | US | 2019247269 | A1 | 15 August 2019 |
| | | | | US | 11071678 | B2 | 27 July 2021 |
| | | | | WO | 2014138504 | A2 | 12 September 2014 |
| | | | | US | 10265237 | B2 | 23 April 2019 |
| | | | | IN | 6046 | CHN2015A | 23 October 2015 |
| | | | | EP | 2964164 | A2 | 13 January 2016 |
| | | | | EP | 2964164 | B1 | 20 May 2020 |
| | | | | US | 2021346235 | A1 | 11 November 2021 |
| | | | | US | 11672726 | B2 | 13 June 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210650783
- CN 112168452 A
- CN 215081530 U
- CN 114041910 A
- CN 109172082 A
- CN 113974937 A
- CN 213310693 U
- CN 210990977 U